# EUROPEAN PATENT SPECIFICATION

(11) **EP 2 748 609 B1**
(45) Date of publication and mention of the grant of the patent: **24.04.2019**
(21) Application number: 12745608.5
(22) Date of filing: 25.07.2012
(51) Int. Cl.: G01N 33/579

(54) **DETECTION OF ENDOTOXIN IN AQUEOUS SOLUTION**
DETEKTION VON ENDOTOXIN IN EINER WÄSSRIGEN LÖSUNG
DÉTECTION D'ENDOTOXINES DANS UNE SOLUTION AQUEUSE

(30) Priority: 01.09.2011 US 201161529943 P
(43) Date of publication of application: 02.07.2014
(73) Proprietor: Fresenius Medical Care Holdings, Inc., Waltham, MA 02451-1457 (US)
(72) Inventor: HENRIE, Michael, Ogden, UT 84404 (US)
(74) Representative: Pulieri, Gianluca Antonio
(86) International application number: PCT/US2012/048036
(87) International publication number: WO 2013/032601

(56) References cited:
- EP-A1- 0 649 021
- EP-A2- 0 347 951
- GB-A- 1 522 127
- JP-A- 2009 085 880
- US-A- 4 276 050
- BATHE ET AL: "Gut is not a source of cytokines in a porcine model of endotoxicosis", SURGERY, C.V. MOSBY CO., ST. LOUIS, US, vol. 120, no. 3, 1 September 1996 (1996-09-01), pages 522-533, XP005441117, ISSN: 0039-6060, DOI: 10.1016/S0039-6060(96)80073-0
- YOUNG N S ET AL: "An invertebrate coagulation system activated by endotoxin: evidence for enzymatic mediation.", THE JOURNAL OF CLINICAL INVESTIGATION JUL 1972 LNKD- PUBMED:4624351, vol. 51, no. 7, July 1972 (1972-07), pages 1790-1797, XP002684767, ISSN: 0021-9738

## Description

### BACKGROUND OF THE INVENTION

The present invention relates to a method of detecting endotoxin in aqueous solution. JP 2009 085880 A and US 4 276 050 A belong to the background art of the present invention, these documents disclosing a known measuring vessel and a known method for systemic endotoxin detection respectively.

Endotoxins are fever-inducing bacterial toxins found in gram-negative bacteria. Gram-negative bacteria are commonly found in aquatic environments. Gram-negative bacteria do not become stained during the Gram staining process, whereas gram-positive bacteria turn purple under the staining technique. Endotoxins can be found within the cell wall of gram-negative bacteria. As the cells grow and die, the endotoxins can be released into the surrounding aquatic environment.

Endotoxins are an ongoing concern in the medical treatment, healthcare, and pharmaceutical fields. Sterilization of devices and solutions using a heating method can kill gram negative bacteria that might be present in the product or solution with the release of endotoxin that can remain in the product. The endotoxin can be heat stable. Exposure to endotoxins associated with gram-negative bacteria can lead to effects such as fever, cytokine system activation, endothelial cell destruction, blood vessel permeability effects, or other adverse effects.

Bacterial endotoxin tests (BETs) were done for many years by the USP Rabbit Pyrogen Test, which is a time consuming and costly animal test. In 1983, the US FDA approved the Limulus Amebocyte Lysate (LAL) test as a standard test for endotoxins. LAL is an *in-vitro* test for gram-negative endotoxin. The LAL test may be substituted for the USP Rabbit Pyrogen Test for end-product testing of human and animal parenteral drugs, biological products, and medical devices, when used according to US FDA Guidelines published in 1987.

The use of LAL for the detection of endotoxin evolved from the observation that the *Limulus polyphemus* (horseshoe crab) is particularly sensitive to endotoxin from gram-negative infection. The white blood cells (*amebocytes*) within the horseshoe crabs were observed to blood clot when exposed to endotoxin. A lysate prepared from washed amebocytes was developed, which was an extremely sensitive indicator of the presence of endotoxin, and which formed a basis of the LAL test. *E*.*g*., Levin, J. and F.B. Bang, (1968) Thrombos. Diath. Haemorrh. 19:186-197. To obtain LAL, horseshoe crabs can be caught, examined for health, and non-lethally bled (e.g., using a stainless steel needle that is inserted into their circulatory system), and then can be returned to their habitat. The crab's extracted blood is centrifuged to separate the suspended amebocytes from the liquid plasma. The amebocytes are then freeze - dried and processed for use.

The general principle of a LAL test is well-understood and has been thoroughly documented in the art. *E*.*g*., Young, N.S. et al., (1972) J. Clin. Investig. 51:1790-1797. Gram-negative bacterial endotoxin catalyzes the activation of a proenzyme in the *Limulus* Amebocyte Lysate. The initial rate of activation is determined by the concentration of endotoxin present. The activated enzyme (coagulase) hydrolyzes specific bonds within a clotting protein (coagulogen) also present in *Limulus* Amebocyte Lysate. Once hydrolyzed, the resultant coagulin self-associates and forms a gelatinous clot that can be detected visually. A LAL test that exploits these reactions is the Gel-Clot method. Another LAL test is the LAL chromogenic method which uses a modified LAL and a synthetic color-producing substrate to detect endotoxin presence. The chromogenic method is quantitative and the color intensity developed upon addition of the sample to the LAL supplied with the kit is proportional to the amount of endotoxin present in the sample and can be calculated from a standard calibration curve. The chromogenic method, however, requires a more specialized reagent and chemistries and the use of an optical detector and curve-based calculation by the user.

LAL has been used to test aqueous samples for endotoxins. In the field of dialysis, for example, the Association for the Advancement of Medical Instrumentation (AAMI) has published standards for maximum allowable concentrations of bacteria and endotoxin in these solutions (endotoxin level should not exceed 2.0 EU/mL as tested by the LAL assay, and action must be taken when the level exceeds 1.0 EU/mL); see, Association for the Advancement of Medical Instrumentation (AAMI), Hemodialysis systems; AAMI RD52-2004, Arlington, Virginia. In a typical dialysis system, blood and dialysate are pumped into a dialyzer from opposite directions. Water typically is supplied through water lines for the preparation of dialysate on a machine and for cleaning/rinsing operational modes performed on the machine between sessions. In addition, water dialysis machines can be offline for significant periods of time between patient sessions or due to regular maintenance. Also, in a dialysis clinic, multiple machines may be supplied water using a shared central water distribution system. Changes may be made to an existing central water distribution, such as the addition of a new distribution line or lines. A dialysis system poses a number of situations where endotoxin testing can be applied. Off-site testing by laboratory assays are known and used which can provide highly accurate assays of aqueous solutions for endotoxins, but require time and expense.

Currently available LAL tests on dialysate include reliance on a gel-clot assay which requires incubation of test vials in an incubator (heating and heat control unit). Previously, if the user wants to have greater control over the sensitivity or shorten the assay time, photometric LAL methods, such as the turbidimetric or the kinetic chromogenic method typically have been used. Both of these photometric methods, however, require specialized technical expertise and a special machine to detect the test results, e.g., a microplate reader. The requirements of photometric LAL methods increase cost.

A rapid, easy, and inexpensive method for detecting the presence of a threshold level of endotoxin in aqueous solutions is needed which does not require incubators for assay vials and which can reduce the need for costly analysis devices or time-consuming and costly laboratory assays.

### SUMMARY OF THE INVENTION

The present description relates to a rapid, simple, and cost-effective test kit for detecting the presence of a threshold level of endotoxin in aqueous samples, such as water for dialysis and dialysate, or other aqueous fluids.

The present description relates to a test kit for detecting the presence of a threshold level of endotoxin in aqueous samples using an endotoxin-activatable clotting agent-based gel clot assay, which can be conducted at ambient temperatures without the need of incubation of sample and control vials.

Furthermore, the present description relates to a test kit to afford a quick and simple test to determine if a water source or distribution system is conforming to standard limits.

A feature of the present invention is to provide a method for readily and inexpensively test aqueous samples for the presence of threshold levels of endotoxin.

Additional features and advantages of the present invention will be set forth in part in the description that follows, and in part will be apparent from the description, or may be learned by practice of the present invention. The features and other advantages of the present invention will be realized and attained by means of the elements and combinations particularly pointed out in the description and appended claims.

To achieve these and other advantages, the present description relates in part to a test kit for detecting endotoxin in an aqueous sample using a gel-clot method. The test kit includes at least one transparent assay container, at least one transparent control container, and dried endotoxin-activatable clotting agent pre-introduced in the assay and control containers or introducible therein by transfer from a clotting agent supply container. The assay container defines an internal compartment that is capable of containing dried endotoxin-activatable clotting agent in combination with a predetermined volume of aqueous sample received in the compartment through an access opening (e.g., reclosable or sealable) of the container. The assay container is manually manipulatable for mixing the dried endotoxin-activatable clotting agent and aqueous sample to form an assay mixture. The dried endotoxin-activatable clotting agent and aqueous sample are capable of forming a gel clot in the assay mixture in a predetermined period of time at ambient temperature when endotoxin is present in the aqueous sample in at least a preselected threshold concentration. The at least one transparent control container is capable of containing a control mixture comprising a defined quantity of the dried endotoxin-activatable clotting agent, which is capable of reacting with a predetermined volume of control standard endotoxin of known endotoxin concentration added to the control container to form a gel clot within the same predetermined period of time as the assay container to validate the results. The assay and control containers holding the respective assay and control mixtures can be stored at an ambient temperature which, for example, can be between the freezing and boiling temperatures of the aqueous sample, during the predetermined period of time allocated for gel clot formation.

The present invention relates to a method of detecting endotoxin in an aqueous sample which includes steps a) - f). In step a), dried endotoxin-activatable clotting agent is combined with a predetermined volume of aqueous sample in a compartment of a closed transparent assay container. The dried endotoxin-activatable clotting agent is pre-introduced in the assay container before the combining with the aqueous sample. The aqueous sample is received in the compartment through a reclosable access opening of the container, such access opening of the assay container comprising a luer lock or a luer slip. In step b), the assay container is manually manipulated to mix the dried endotoxin-activatable clotting agent with a predetermined volume of aqueous solution to form an assay mixture in the compartment. As indicated, the dried endotoxin-activatable clotting agent and aqueous sample are capable of forming a gel clot in the assay mixture in a predetermined period of time at ambient temperature when endotoxin is present in the aqueous sample in at least a preselected threshold concentration. In step c), control standard endotoxin of known endotoxin concentration is mixed with a defined quantity of the dried endotoxin-activatable clotting agent in a transparent control container to form a positive control mixture. The control standard endotoxin is added in a precalculated volume to form a concentration of the endotoxin in the positive control mixture that is effective for gel clot formation within the predetermined period of time. In step d), the assay container containing the assay mixture of step b) with the access opening closed and the positive control containing the positive control mixture of step c) are stored at an ambient temperature of from about 18°C to about 29°C for the predetermined period of time. The assay container is left undisturbed during the predetermined period of time of the storing. In step e), the assay container is inverted when the predetermined period of time ends and a visual determination is made on whether any gel clot which is present at an upper end of the compartment remains at the upper end of the container for a preselected period of time. In step f), the control container is inverted when the predetermined period of time ends and visually checked for a gel clot in a similar way as the assay container to validate the results.

It is to be understood that both the foregoing general description and the following detailed description are exemplary and explanatory only and are only intended to provide a further explanation of the present invention, as claimed.

The accompanying drawings, which are incorporated in and constitute a part of this application, illustrate some of the embodiments of the present invention and together with the description, serve to explain the principles of the present invention. The drawings are not necessarily drawn to scale. Like numerals in the drawings refer to like elements in the various views.

### BRIEF DESCRIPTION OF THE DRAWINGS

FIG. 1 shows an elevation view of an assay container and disposable endotoxin-free aqueous sample transfer instrument of a test kit according to an example of the present description.
FIG. 2 shows the test container of FIG. 1 including volumetric markings according to examples of the present description.
FIG. 3 shows the test container of FIG. 1 after mixing a lyophilized endotoxin-activatable clotting agent and aqueous solution in the assay container according to an example of the present description.
FIG. 4A shows a positive (gel clot) reaction result at the right side and a negative (aqueous solution) reaction result at the left side, and FIG. 4B shows a positive Control Sample which shows a positive (gel clot) reaction result to validate the results, according to an example of the present description.

### DETAILED DESCRIPTION OF THE PRESENT INVENTION

The present description relates to an endotoxin sampling and detection kit which can provide an efficient, rapid, and accurate way to determine if endotoxin is present in an aqueous sample at a level that exceeds a selected threshold level (concentration) value. The assay can be conducted at ambient temperature without the need to use an incubator for assay or control containers (e.g., test vials, test tubes, and the like). As used herein, the "ambient temperature" is the environmental temperature of the room airspace or surrounding air in which the testing is conducted. Ambient temperature is free of direct thermal heating applied to the assay and control containers by an incubator device or system. The ambient temperature for testing can be, for example, any temperature between the freezing and boiling temperatures of the aqueous sample, or from about 65°F to about 85° F (from about 18°C to about 29° C), or about 68°F to about 82° F, or about 71°F to about 79° F, or other temperatures. As an option, the ambient temperature can be a room temperature to which all of the assay and control container reagents have been permitted to equilibrate before starting the assay and at which the assay and control mixtures are held until the test endpoint. As an option, the ambient temperature can be fixed or permitted to drift within any of these temperature ranges during an assay, and still provide reliable results. The results of the assay are capable of being evaluated visually without need for photometric detectors or other analytical devices. The assay can permit rapid pre-screening of fluids for threshold levels of endotoxin that may be present in an aqueous sample before resorting to more time-consuming and costly laboratory assays on the aqueous fluid of interest. As an option, the test kits of the present description can be used in a dialysis clinic or center as part of endotoxin surveillance procedures for the supply water, distribution line water that supplies one or more dialysis machines, dialysis treatment solutions used with a dialysis machine (e.g., dialysates), or any combinations of these. New water distribution lines that are being brought online or dialysis machines have been offline are included without limitation as sites where water testing for endotoxins with the test kits of the present description can be used.

The endotoxin sampling and detection kit can include at least one assay container and at least one positive control container. The endotoxin, which can be detected, can be a gram-negative bacterial endotoxin. For example, the endotoxin can be a molecular component of *Escherichia coli* (*E. coli*), *Klebsiella* species, *Enterobacter* species, *Citrobacter* species, *Pseudomonas* species, *Acinetobacter* species, *Serratia* species, *Morganella* species, *Alcaligenes* species, or others. The molecular component can be, for example, a lipopolysaccharide (LPS), which can be bound to a protein and phospholipid. The LPS can be located on the outer membrane of gram negative bacteria. Gram-negative bacterial endotoxin present in an aqueous sample can catalyze the activation of a cascade of biochemical reactions in the presence of an endotoxin-activatable clotting agent introduced into the assay container, which culminate in the formation of a solid gelatinous clot in the container. A control standard endotoxin added to the control container catalyzes similar biochemical reactions in the same clotting agent added therein to lead to a similar result. The time period required for the gel clot to form is a function of the level of endotoxin in the aqueous sample for the assay container, and the endotoxin level of the control standard endotoxin for the control container. This endotoxin level or concentration of the aqueous sample or control standard endotoxin can be expressed in units of EU/mL. As used herein, "EU" refers to Endotoxin Unit. One (1) EU for United States Pharmacopoeia (USP) is equivalent to one (1) International Unit (IU), and values in EU herein can be equally reported in IU and vice versa (i.e., 1 EU = 1 IU). Increasingly higher endotoxin concentrations in an aqueous sample (or standard), for example, are correlated to increasingly faster gel clot times and, conversely, increasingly lower endotoxin concentrations in an aqueous sample (or standard) are correlated with increasingly slower gel clot times. These gel clot times generally can be insensitive to the amount of endotoxin-activatable clotting agent when used in formats of the present invention. As an option, a sufficient amount of endotoxin-activatable clotting agent is used to generate an easily visible clot in the presence of endotoxin without need of optical magnification, and is small enough to be applied in relatively small capacity test containers (e.g., vials with a capacity less than about 25 mL, or smaller or larger volumes). As another option, at least one disposable endotoxin-free fluid transfer instrument can be included with the test kit. A fluid transfer instrument can be used for transferring aqueous samples of an aqueous fluid of interest to the assay container. A fluid transfer instrument can be used to transfer control standard endotoxin to a control container, or other uses which may be associated with the test kit or components thereof such as indicated herein. Fluid transfer instruments should be used for one such fluid transfer operation and then disposed without reuse to reduce risk of cross-contaminations. A plurality of separate fluid transfer instruments can be used for a single assay.

As an option, the assay and positive control tests can be run concurrently for a common predetermined period of time for which gel clotting is correlated with a minimum or threshold level or concentration of endotoxin in water. In conducting the assay with the kit, a sample of aqueous solution of interest and endotoxin-activatable clotting agent are combined and mixed in the assay container. For the positive control, a specific volume of a control standard endotoxin containing at least the same or more than the indicated threshold concentration (e.g., twice) of endotoxin to be evaluated for in the assay container is added to the control container. At the end of the predetermined period of time after mixing the reagents of the assay and control containers, the assay container and control container each can be inverted and checked for a gel clot. The presence of gel clot in the assay container that remains intact for at least a brief period of time after the container is inverted (e.g., for at least about 30 seconds, or for at least about 20 seconds, or for at least about 15 seconds, or other non-momentary times), is a positive (+) result that indicates the presence of endotoxin in the aqueous sample in at least a threshold level. The absence of a gel clot is a negative (-) result indicating that the level of any endotoxin present in the aqueous sample is below the preselected threshold level. The lysate may show an increase in turbidity or viscosity without a visible appearance of an intact gel clot that persists for at least the indicated brief period of time, which is considered a negative result. The presence of a gel clot in the control container validates the assay results. The absence of a gel clot in the control container means the assay results are invalid, and the test should be repeated.

As an option, the control standard endotoxin comprises a specified bacteria (e.g., *E. coli*) in a specified concentration in a water medium. The control standard endotoxin can be prepared, for example, by reconstituting (rehydrating) dried (e.g., lyophilized, i.e., freeze-dried) endotoxin of a known or precertified potency in endotoxin-free water of a measured volume, vortexing the mix for at least about 5 minutes after rehydration, and for at least about one minute immediately prior to each use. The dried endotoxin can be supplied in vial, for example, as a purified lipopolysaccharide (LPS), which is freeze-dried in a stabilized matrix. As an option, a two-fold dilution series can be used in preparing the positive control. For two-fold dilution, for example, the sensitivity of the control standard endotoxin is prepared to be twice that of the threshold endotoxin concentration that has been selected for the evaluation of the aqueous samples of unknown endotoxin content to be assayed. For example, for an assay using a selected threshold endotoxin concentration of 5 EU/mL for the aqueous samples (unknowns), where lyophilized endotoxin is obtained for use that has a certified potency of 220 EU/vial (e.g., 22 EU/ng for a 10 ng-containing vial) for purposes of this illustration, the lyophilized endotoxin material can be reconstituted with 22 mL of endotoxin-free water (e.g., LAL reagent grade water) to obtain a 10.0 EU/mL control standard endotoxin that can be used in the control containers of the test kit. Other dilution factors that may be used in the preparation of the control standard endotoxin can be calculated in a similar manner. A series of dilution factors for the dried (e.g., lyophilized) endotoxin material also can be provided on printed instructions included with the kit to assist the user, which dilution factors are correlated to various corresponding endotoxin concentrations. As an option, a plurality of the vials of dried (e.g., lyophilized) endotoxin of a known or precertified potency by the commercial supplier can be included or supplied with the kit. Alternatively, the vials of dried (e.g., lyophilized) endotoxin of a known or precertified potency can be obtained by the kit user from the commercial supplier. As an option, the rehydrated endotoxin material can be stored at 2-8°C for up to about four weeks before use, and the lyophilized endotoxin material can be stored before rehydration and use at controlled room temperature or refrigerated.

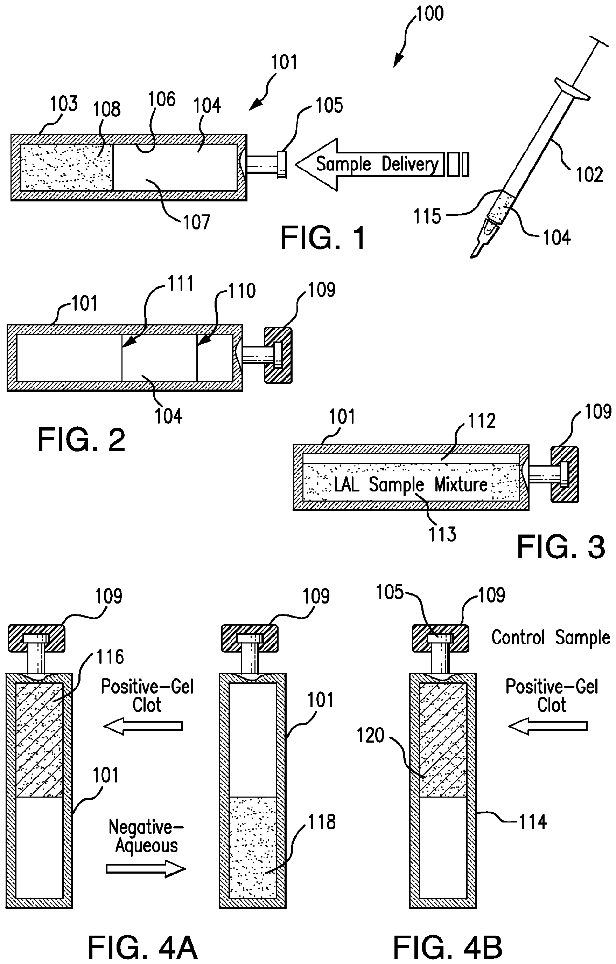

FIG. 1 shows one example of an assay test kit 100 including an assay container 101 and a disposable endotoxin-free fluid transfer instrument 102 according to an example of the present description. The assay container 101 can comprise a transparent housing 103. The housing can be, for example, a plastic or glass housing. A plastic housing can be, for example, polycarbonate, polystyrene, polymethacrylate, or other polymers, which can be molded into the shape of the container. The transparent housing 103 can be constructed of inexpensive material. The housing can be "transparent" by allowing sufficient light to pass through so that objects within the housing, such as a gel clot and aqueous solution, can be distinctly visually seen and distinguished from each other from outside the container. The housing can have one part (e.g., window) that is visible to a person to determine clotting or not, and this is considered "transparent" for purposes of the present invention. The housing can have any shape or size. The assay container can be provided in manually-manipulatable sizes. The assay container can be vial, tube, or other handheld receptacle. The assay container can have dimensions, for example, of from about 7 mm to about 20 mm in diameter and from about 25 mm to about 75 mm in length, or from about 12 mm to about 15 mm in diameter and from about 40 mm to about 50 mm in length, or other dimensions. The housing 103 of the assay container 101 has an internal compartment 104 that comprises internal walls 106, which define an enclosed space 107. The compartment 104 can be sized to accommodate a defined amount of dried (e.g., lyophilized) endotoxin-activatable clotting agent 108 used for the measurement of endotoxin levels in aqueous samples and the volume of test sample itself that is introduced into the assay container 101. The endotoxin-activatable clotting agent 108 is illustrated as lyophilized LAL reagent in FIGS. 1 and 3, but is not limited thereto. The container 101 can be fitted with an access 105 on one end, for example, or elsewhere for introducing the aqueous sample into the assay container 101. A biomedical connector can be used for the access 105, such as a luer lock or luer slip or other biomedical connector. The access 105 can include an overcap 109, such as a screwable cap. The overcap 109 can be used for opening the container 101 for introduction of the sample and any lyophilized LAL reagent if not already contained through access 105, and closing the access 105 and container in a fluid-tight manner after introduction of the aqueous sample and before mixing the aqueous sample with the lyophilized LAL reagent therein. An overcap 109 for the access 105 is shown in FIGS. 2, 3, and 4A. A positive control container of the test kit can have similar housing and access structures and features as the assay container 101, or it can be different. FIG. 4B shows a control container 114 with an access 105 and cap 119. As an option, the housing, access, and cap of the control container 114 can be similar structures and components as those indicated for the assay container 101. As indicated, the control container 114 differs from assay container 101, wherein the control container receives control standard endotoxin of known endotoxin concentration, whereas the assay container 101 receives the aqueous sample of unknown endotoxin concentration. The control container 114 also can be varied from the assay container 101 by use of a different colored cap 119 from cap 109 of the assay container, or another visually detectible identifier on the caps or containers used to distinguish the assay and control containers from each and avoid possible confusion when the assay results are evaluated.

As an option, the endotoxin-activatable clotting agent can be lyophilized horseshoe crab amebocyte lysate. As an option, lyophilized amebocyte lysates derived from the taxonomic family *Limulidae* can be used for the endotoxin-activatable clotting agent. This taxonomic family includes the species of *Limulus polyphemus*, which is commonly referred to as the Atlantic horseshoe crab, and *Tachypleus tridentatus*, which is commonly referred to as the East Asian horseshoe crab, *Carcinoscorpius rotundicauda*, which is commonly referred to as the mangrove horseshoe crab found in Southeast Asia, and *Tachypleus gigas* , which is another species of horseshoe crab found in South and Southeast Asia. The endotoxin-activatable clotting agent 108 can be, for example, a lyophilized *Limulus* amebocyte lysate (LAL reagent) in freeze-dried powder or pellet form. Alternatives to LAL can include, for example, *Tachypleus* amebocyte lysate (TAL) in freeze-dried or pellet form, which can function similarly to LAL in detection of gram-negative bacterial endotoxin. The present invention can encompass any cloned forms of the toxin-detecting gene in horseshoe crab blood. Further, lipopolysaccharide (LPS)-binding proteins of freshwater crayfish, silkworm larvae, mollusks, and gastropods, which can support endotoxin-induced protein coagulation reactions also may be suitable alternatives to LAL. The sensitivity of a LAL to an endotoxin, for example, typically is lot specific. Commercial suppliers of LAL often provide certifications of the sensitivity of a particular lot, which can be expressed in units of EU/mL sensitivity for a specified bacterial strain (e.g., a strain of *E. coli*).

In FIG. 1, a sample of aqueous solution to be tested with the test kit 100 can be transferred to the assay container 101 with a syringe or pipette or other endotoxin-free fluid transfer instrument 102. For example, sterile, individually wrapped plastic or glass pipettes and pipette tips which are endotoxin-free can be used. Mechanical pipetters with removal tips may be used, so long as they are endotoxin-free. A plurality of the disposable endotoxin-free fluid transfer instruments can be included with the test kit, or can be provided otherwise, such as by the user as obtained from commercial sources.

The assay container, fluid transfer instrument, or both can be designed to introduce an aqueous sample into the compartment of the assay container in a volumetrically measured amount. FIG. 2 shows assay container 101 of the test kit including several options of visually identifiable volumetric markings 110 and 111 according to examples of the present description. As an option, dried (e.g., lyophilized) endotoxin-activatable clotting agent can be pre-arranged in the compartment of the assay container as supplied with the kit. In this option, a visible marking 110 can be provided on the container that corresponds to a desired total volume of a mixture of the endotoxin-activatable clotting agent as combined with an aqueous sample solution to be assayed. The endotoxin-activatable clotting agent already in the container dissolves in the added aqueous solution upon mixing. For this option where the endotoxin-activatable clotting agent is pre-arranged in the test container, a proper marking location for containers of a given compartment geometry can be readily determined by use of empirical testing and observation in the design and manufacture of the given type of container. For example, known volumes of the clotting agent (in solid form) and water can be introduced and mixed in a container having a compartment of known volume and geometry, and the location of the surface of the resulting mixture (which includes the dissolved endotoxin-activatable clotting agent and aqueous fluid) with the container in an upright position can be marked on the assay container. This surface location 110 marking reflects the total volume of this initial mixture. In this option, aqueous sample can be added to an assay container which already contains a defined quantity of endotoxin-activatable clotting agent until visible marking 110 on the container is reached, which corresponds to the predetermined total volume. The volume of aqueous sample introduced for these containers can be a standard or an essentially constant value. The volume or amount of endotoxin-activatable clotting agent introduced first into the container can be based on the lot and can be pre-determined based on the lot's sensitivity. The volume of aqueous sample is the difference between the marked total volume and the known volume of the endotoxin-activatable clotting agent added to the container. As an option, the combined volume of the mixture of aqueous solution and endotoxin-activatable clotting agent is less than the total volumetric capacity defined by the compartment 104, and thus the mixture does not totally fill the compartment 104. As shown in FIG. 3, this can leave an empty head space 112 within the container 101, which can assist in the mixing of the mixture 113 comprising the combined sample and endotoxin-activatable clotting agent components.

As shown in FIG. 2, a visually identifiable volumetric marking 111 can be provided on the container 101, which corresponds to a predetermined volume of aqueous solution that is present alone in the compartment 104 of container 101. This can be used, for example, where the test container 101 is initially empty, and the aqueous sample is added first to the container compartment 104, such as up to the marking 111 on the container, before the dried endotoxin-activatable clotting agent is added in a predetermined quantity or volume of its own. In this option, when the dried endotoxin-activatable clotting agent is added after the introduction of the aqueous solution introduction to the container, the volume of the dried clotting agent can be controlled by use of a dry powder measuring transfer device or by the predetermining the volume or amount of a larger solid discrete form thereof if used (e.g., a pellet or pellets).

As shown in FIG. 1, a visually identifiable volumetric marking 115 can be provided on a disposable endotoxin-free fluid transfer instrument 102, such as a syringe or pipette, used to transfer the aqueous sample solution to the assay container 101 of the test kit 100. The visible marking 115 on the fluid transfer instrument 102 can be used to ensure that a prespecified volume of aqueous sample solution is introduced in the compartment 104 of the assay container 101. As an option, graduated pipettes or syringes which have a series of marked lines indicating different corresponding volumes can be used.

FIG. 3 shows the assay container 101 of FIG. 1 after mixing an endotoxin-activatable clotting agent and aqueous sample in the assay container 101 to provide an assay mixture 113 according to an example of the present application. The assay mixture 113 can have a mixing ratio of the endotoxin-activatable clotting agent to the predetermined volume of the aqueous sample of from about 1:1 to about 1:5, or from about 1:2 to about 1:4, or from about 1.25:2 to about 1:3.5, all on a mL:mL basis, or other mixing ratios. The assay mixture 113 can have a total volume of from about 1 mL to about 10 mL, or from about 1 mL to about 4 mL, or from about 1.5 mL to about 3.5 mL, or from about 2.0 mL to about 3.0 mL, or other values above, below, or within any of these ranges. As indicated, the access connector 109 can be closed after the aqueous sample is introduced in the compartment of the container. The assay container can be sized and configured to be manually manipulatable to allow the aqueous sample to be mixed with the endotoxin-activatable clotting agent, thus initiating the biochemical reaction. To ensure adequate mixing, the assay container 101 can be gently inverted, or tilted and gently swirled, or other manual manipulations, until the contents are in solution. The assay container can then be placed in a holder or in a storage location, e.g., a storage compartment, or essentially anywhere, and left undisturbed for a specific period of time, after which it is observed for gel clotting. Any convenient accurate timer which shows at least minute time can be used to monitor the time period between the initiation of mixing of indicated components in the assay container and the time of checking for gel clotting. The assay reaction can be such that if the endotoxin level in the sample exceeds a specified limit or threshold value, then the mixture in the assay container will include a gel clot at the end of the predetermined time period, e.g., a time period less than about 60 minutes, or a time period less than about 30 minutes, or a time period in the range of from about 10 to about 25 minutes, or from about 10 to about 20 minutes, or from about 15 to about 20 minutes, or other time periods. For example, based on the predetermined sensitivity of an endotoxin-activatable clotting agent of a particular lot as certified by the supplier, the gel clot time can be predetermined for that sensitivity of that lot and then used to test aqueous samples of unknown endotoxin levels. As an option, the predetermined time period allowed for gel clot formation after mixing can have a tolerance of ± 2 minutes, or ± 1 minute, or other selected tolerance.

As an option, the endotoxin-activatable clotting agent used in the assay can have a sensitivity to a specified endotoxin of from about 0.5 to about 5.0 EU/mL, such as 0.5 EU/mL, or 1.0 EU/mL, or 1.5 EU/mL, or 2.0 EU/mL, or 2.5 EU/m, or 3.0 EU/mL, or 3.5 EU/mL, or 4.0 EU/mL, or 4.5 EU/mL, or 5.0 EU/mL, or other values of sensitivity depending on the time of the test (i.e., the predetermined period of time between mixing the endotoxin-activatable clotting agent and aqueous sample and the checking for a gel clot).

As indicated, the biochemical reactions in the assay and control containers of the test kit are time sensitive. If the assay mixture is in a gel clot state at the end of the predetermined time period, then the endotoxin concentration of the sample at least meets the specified threshold limit. If the assay mixture is not in a gel clot state at the predetermined time, then the endotoxin concentration of the sample is below the specified threshold limit. For example, FIG. 4A, at the left side, shows a positive (gel clot) reaction result in which an intact solid gel clot 116 is present at the upper end of assay container 101 after it is inverted at the endpoint of the predetermined period of time. FIG. 4A, at the right side, shows a negative (aqueous solution) reaction result wherein aqueous solution 118 is present in the bottom of the container 101 after it is inverted. As indicated, the positive control can be used to validate the results. FIG. 4B shows a positive Control Sample which shows a positive (gel clot) reaction result to validate the results, wherein an intact solid gel clot 120 is present at the upper end of the positive control container 114 after it is inverted at the endpoint of the predetermined period of time. Ultimately, any sample mixed with a lyophilized endotoxin-activatable clotting agents, such as a LAL or TAL reagent, can produce a gel clot so that the assay container can be calibrated either using an external standard or calibrated during manufacture. As indicated, the positive control tests can be conducted simultaneously with the assays test to validate the assay results in a concurrent time frame. The test kit of the present description affords a quick and simple test to determine if a water source or distribution system is conforming to standard limits (e.g., AAMI limits) or other selected or applicable limits. The test kit can be used as an initial screening test to determine if standard laboratory endotoxin testing is needed. The use of the screening test can reduce the need, and associated time and costs, of standard laboratory testing time as such testing can be limited to positive samples identified with the test kit of the present description.

For purposes of the present invention, the positive control container and its use are essential.

As an option, the sampled solution can be, for example, water for dialysis or dialysate. The term "dialysate" can include aqueous salt solutions used in the actual dialysis process. To check the water quality of a specific clinic distribution system, dialysis machine, or other source of water, the endotoxin sampling and detection vial can be used to rapidly determine if the water or dialysate has exceeded the allowable limits for endotoxin for the given sample type (e.g., water, dialysate).

Exemplary test kit instructions for detecting endotoxin in an aqueous sample using a lyophilized LAL reagent are illustrated below:
Step 1: Collect an aqueous sample with a disposable endotoxin-free fluid transfer instrument.
Step 2: Aseptically add a measured or measurable volume of aqueous sample into an assay container which can already contain lyophilized LAL reagent (or *vice versa*), cap, and gently mix.
Step 3: Aseptically add a measured amount of control standard endotoxin into a positive control container which already contains lyophilized LAL reagent (or *vice versa*), cap, and gently mix.
Step 4: Store the capped assay and control containers undisturbed at ambient temperature (e.g., 65°F to 85°F) for a predetermined period of time, for instance, using a timer.
Step 5: When the predetermined period of time has ended, invert the assay container and optional control container, for instance, 180 degrees.
Step 6: View and/or record results: positive (+) if a solid gel clot forms for at least about 30 seconds (other times may be used), negative (-) if no solid gel clot forms; and preferably a solid gel clot is required for the positive control container for test to be valid.

The present description can include any combination of these various features or embodiments above and/or below as set forth in sentences and/or paragraphs. Any combination of disclosed features herein is considered part of the present description and no limitation is intended with respect to combinable features.

Further, when an amount, concentration, or other value or parameter is given as either a range, preferred range, or a list of upper preferable values and lower preferable values, this is to be understood as specifically disclosing all ranges formed from any pair of any upper range limit or preferred value and any lower range limit or preferred value, regardless of whether ranges are separately disclosed. Where a range of numerical values is recited herein, unless otherwise stated, the range is intended to include the endpoints thereof, and all integers and fractions within the range. It is not intended that the scope of the invention be limited to the specific values recited when defining a range.

It will be apparent to those skilled in the art that various modifications and variations can be made to the embodiments of the present invention without departing from the scope of the present invention. Thus, it is intended that the present invention covers other modifications and variations of this invention provided they come within the scope of the appended claims.

## Claims

1. A method of detecting endotoxin in aqueous solution comprising:
a) combining dried endotoxin-activatable clotting agent with a predetermined volume of aqueous sample in a compartment of a closed transparent assay container, wherein the dried endotoxin-activatable clotting agent is pre-introduced in the assay container before the combining with the aqueous sample, wherein the aqueous sample is received in the compartment through a reclosable access opening of the assay container, wherein the access opening of the assay container comprises a luer lock or a luer slip;
b) manually manipulating the assay container to mix the dried endotoxin-activatable clotting agent with the predetermined volume of aqueous solution in the compartment to form an assay mixture, wherein the dried endotoxin-activatable clotting agent and aqueous sample are capable of forming a gel clot in the assay mixture in a predetermined period of time at ambient temperature when endotoxin is present in the aqueous sample in at least a preselected threshold concentration;
c) mixing control standard endotoxin of known endotoxin concentration with a defined quantity of the dried endotoxin-activatable clotting agent in a transparent control container to form a positive control mixture, wherein the control standard endotoxin is added in a precalculated volume to form a concentration of the endotoxin in the positive control mixture that is effective for gel clot formation within the predetermined period of time;
d) storing the assay container containing the assay mixture of step b) with the access opening closed and the positive control container containing the positive control mixture of step c) at an ambient temperature of from about 18°C to about 29°C for the predetermined period of time, wherein the assay container is left undisturbed during the predetermined period of time of the storing;
e) inverting the assay container when the predetermined period of time ends and visually determining if any gel clot which is present at an upper end of the compartment remains at the upper end of the assay container for a preselected period of time; and
f) inverting the control container when the predetermined period of time ends and visually checking for a gel clot to validate the results.

2. The method of claim 1, wherein the preselected period of time for the gel clot to remain visible is at least about 30 seconds.

3. The method of claim 1, wherein endotoxin-activatable clotting agent is horseshoe crab amebocyte lysate or dried Limulus amebocyte lysate (LAL) or is dried Tachypleus amebocyte lysate (TAL).

4. The method of claim 1, wherein endotoxin-activatable clotting agent has a sensitivity to the endotoxin of from about 0.5 to about 5.0 EU/mL for the predetermined period of time.

5. The method of claim 1, wherein the aqueous solution is water for dialysis or dialysate.

6. The method of claim 1, wherein the assay mixture has a mixing ratio of the endotoxin-activatable clotting agent to the predetermined volume of the aqueous sample of from about 1:2 to about 1:4 on a mL:mL basis.

7. The method of claim 1, wherein the predetermined time period is less than about 60 minutes, such as from about 10 to about 20 minutes.

## Patentansprüche

1. Verfahren zum Nachweis von Endotoxin in wässriger Lösung, umfassend:
a) Kombinieren von getrocknetem, durch Endotoxin aktivierbarem Gerinnungsmittel mit einem vorbestimmten Volumen einer wässrigen Probe in einer Kammer eines geschlossenen, transparenten Assaybehälters, wobei das getrocknete, durch Endotoxin aktivierbare Gerinnungsmittel vor dem Kombinieren mit der wässrigen Probe in den Assaybehälter eingebracht wird, wobei die wässrige Probe in der Kammer durch eine wiederverschließbare Zugangsöffnung des Assaybehälters empfangen wird, wobei die Zugangsöffnung des Assaybehälters ein Luer-Lock oder ein Luer-Slip umfasst;
b) manuelles Manipulieren des Assaybehälters, um das getrocknete, durch Endotoxin aktivierbare Gerinnungsmittel mit dem vorbestimmten Volumen der wässrigen Lösung in der Kammer zu mischen, um ein Assaygemisch zu bilden, wobei das getrocknete, durch Endotoxin aktivierbare Gerinnungsmittel und die wässrige Probe befähigt sind, bei Umgebungstemperatur in einer vorbestimmten Zeitspanne ein Gelkoagulat in dem Assaygemisch zu bilden, wenn Endotoxin zumindest in einer vorab ausgewählten Schwellenkonzentration in der wässrigen Probe vorhanden ist;
c) Mischen eines Kontroll-Standardendotoxins von bekannter Endotoxinkonzentration mit einer definierten Menge des getrockneten, durch Endotoxin aktivierbaren Gerinnungsmittels in einem transparenten Kontrollbehälter, um ein positives Kontrollgemisch zu bilden, wobei das Kontroll-Standardendotoxin in einem vorab berechneten Volumen zugegeben wird, um eine Konzentration des Endotoxins in dem positiven Kontrollgemisch zu bilden, die innerhalb einer vorbestimmten Zeitspanne wirksam für die Bildung eines Gelkoagulats ist;
d) Aufbewahren des Assaybehälters, der das Assaygemisch aus Schritt b) enthält, mit geschlossener Zugangsöffnung, und des Positivkontrollbehälters, der das positive Kontrollgemisch aus Schritt c) enthält, bei einer Umgebungstemperatur von etwa 18°C bis etwa 29°C für die vorbestimmte Zeitspanne, wobei der Assaybehälter während der vorbestimmten Zeitspanne der Aufbewahrung störungsfrei belassen wird;
e) Umdrehen des Assaybehälters, wenn die vorbestimmte Zeitspanne endet, und visuelle Bestimmung, ob ein jegliches Gelkoagulat, das an einem oberen Ende der Kammer vorhanden ist, für eine vorab gewählte Zeitspanne am oberen Ende verbleibt; und
f) Umdrehen des Kontrollbehälters, wenn die vorbestimmte Zeitspanne endet, und visuelles Prüfen auf ein Gelkoagulat, um die Ergebnisse zu validieren.

2. Verfahren nach Anspruch 1, wobei die vorab gewählte Zeitspanne, für die das Gelkoagulat sichtbar bleibt, mindestens etwa 30 Sekunden beträgt.

3. Verfahren nach Anspruch 1, wobei das durch Endotoxin aktivierbare Gerinnungsmittel Pfeilschwanzkrebs-Amöbozytenlysat oder getrocknetes *Limulus*-Amöbozytenlysat (LAL) oder getrocknetes *Tachypleus-*Amöbozytenlysat (TAL) ist.

4. Verfahren nach Anspruch 1, wobei das durch Endotoxin aktivierbare Gerinnungsmittel eine Empfindlichkeit gegenüber dem Endotoxin von etwa 0,5 bis etwa 5,0 EU/mL für den vorbestimmten Zeitraum aufweist.

5. Verfahren nach Anspruch 1, wobei die wässrige Lösung Wasser für die Dialyse oder Dialysat ist.

6. Verfahren nach Anspruch 1, wobei das Assaygemisch ein Mischungsverhältnis des durch Endotoxin aktivierbaren Gerinnungsmittels zu dem vorbestimmten Volumen der wässrigen Probe von etwa 1:2 bis etwa 1:4, auf mL:mL-Basis, aufweist.

7. Verfahren nach Anspruch 1, wobei die vorbestimmte Zeitspanne weniger als etwa 60 Minuten, beispielsweise etwa 10 bis etwa 20 Minuten, beträgt.

## Revendications

1. Procédé de détection d'une endotoxine dans une solution aqueuse comprenant :
a) la combinaison d'un agent de coagulation sec activable par une endotoxine avec un volume prédéterminé d'échantillon aqueux dans un compartiment d'un récipient d'essai transparent fermé, dans lequel l'agent de coagulation sec activable par une endotoxine est préalablement introduit dans le récipient d'essai avant la combinaison avec l'échantillon aqueux, dans lequel l'échantillon aqueux est reçu dans le compartiment à travers une ouverture d'accès refermable du récipient d'essai, dans lequel l'ouverture d'accès du récipient d'essai comprend un verrouillage Luer ou un glissement Luer ;
b) la manipulation manuelle du récipient d'essai pour mélanger l'agent de coagulation sec activable par une endotoxine avec le volume prédéterminé de solution aqueuse dans le compartiment pour former un mélange d'essai, dans lequel l'agent de coagulation sec activable par une endotoxine et l'échantillon aqueux sont capables de former un caillot de gel dans le mélange d'essai en une période de temps prédéterminée à température ambiante quand une endotoxine est présente dans l'échantillon aqueux à au moins une concentration seuil présélectionnée ;
c) le mélange d'une endotoxine étalon témoin de concentration en endotoxine connue avec une quantité définie de l'agent de coagulation sec activable par une endotoxine dans un récipient témoin transparent pour former un mélange témoin positif, dans lequel l'endotoxine étalon témoin est ajoutée en un volume précalculé pour former une concentration de l'endotoxine dans le mélange témoin positif qui est efficace pour la formation d'un caillot de gel dans la période de temps prédéterminée ;
d) le stockage du récipient d'essai contenant le mélange d'essai de l'étape b) avec l'ouverture d'accès fermée et du récipient témoin positif contenant le mélange témoin positif de l'étape c) à une température ambiante d'environ 18 °C à environ 29 °C pendant la période de temps prédéterminée, dans lequel le récipient d'essai est laissé au repos pendant la période de temps prédéterminée de stockage ;
e) le retournement du récipient d'essai à la fin de la période de temps prédéterminée et la détermination visuelle de si un quelconque caillot de gel qui est présent au niveau d'une extrémité supérieure du compartiment reste au niveau de l'extrémité supérieure du récipient d'essai pendant une période de temps présélectionnée ; et
f) le retournement du récipient témoin à la fin de la période de temps prédéterminée et la vérification visuelle de la présence d'un caillot de gel pour valider les résultats.

2. Procédé selon la revendication 1, dans lequel la période de temps présélectionnée pour que le caillot de gel reste visible est d'au moins environ 30 secondes.

3. Procédé selon la revendication 1, dans lequel l'agent de coagulation activable par une endotoxine est un lysat d'amoebocyte de crabe fer à cheval ou un lysat d'amoebocyte de limule (LAL) sec ou est un lysat d'amoebocyte de Tachypleus (TAL) sec.

4. Procédé selon la revendication 1, dans lequel l'agent de coagulation activable par une endotoxine présente une sensibilité à l'endotoxine d'environ 0,5 à environ 5,0 UE/ml pour la période de temps prédéterminée.

5. Procédé selon la revendication 1, dans lequel la solution aqueuse est de l'eau pour dialyse ou un dialysat.

6. Procédé selon la revendication 1, dans lequel le mélange d'essai présente un rapport de mélange de l'agent de coagulation activable par une endotoxine au volume prédéterminé de l'échantillon aqueux d'environ 1 : 2 à environ 1 : 4 sur une base ml : ml.

7. Procédé selon la revendication 1, dans lequel la période de temps prédéterminée est inférieure à environ 60 minutes, comme d'environ 10 à environ 20 minutes.
